# EUROPÄISCHE PATENTSCHRIFT

(11) **EP 1 360 502 B1**
(45) Veröffentlichungstag und Bekanntmachung des Hinweises auf die Patenterteilung: **01.02.2006**
(21) Anmeldenummer: 02701272.3
(22) Anmeldetag: 12.02.2002
(51) Int. Cl.: G01N 33/68

(54) **LIGANDEN FÜR DEN NACHWEIS VON PRIONEN**
LIGANDS USED FOR DETECTING PRIONS
LIGANDS PERMETTANT LA DETECTION DE PRIONS

(30) Priorität: 13.02.2001 DE 10107083
(43) Veröffentlichungstag der Anmeldung: 12.11.2003
(73) Patentinhaber: Kiesewetter, Holger, 13465 Berlin (DE); Salama, Abdulgabar, 13467 Berlin (DE)
(72) Erfinder: Kiesewetter, Holger, 13465 Berlin (DE); Salama, Abdulgabar, 13467 Berlin (DE)
(74) Vertreter: Luderschmidt, Schüler & Partner GbR
(86) Internationale Anmeldenummer: PCT/EP2002/001451
(87) Internationale Veröffentlichungsnummer: WO 2002/065133

(56) Entgegenhaltungen:
- WO-A-00/29850
- DE-A- 19 730 132
- US-A- 6 033 907
- CAUGHEY B ET AL: "BINDING OF THE PROTEASE-SENSITIVE FORM OF PRION PROTEIN PRP TO SULFATED GLYCOSAMINOGLYCAN AND CONGO RED" JOURNAL OF VIROLOGY, THE AMERICAN SOCIETY FOR MICROBIOLOGY, US, Bd. 68, Nr. 4, 1. April 1994 (1994-04-01), Seiten 2135-2141, XP002056076 ISSN: 0022-538X
- BRIMACOMBE DEBBIE B ET AL: "Characterization and polyanion-binding properties of purified recombinant prion protein." BIOCHEMICAL JOURNAL, Bd. 342, Nr. 3, Seiten 605-613, XP002214909 ISSN: 0264-6021

## Beschreibung

Die vorliegende Erfindung betrifft die Verwendung Liganden zum Nachweis von Prionen, insbesondere im Rahmen eines BSE-Schnelltests. Weiterhin werden Substanzen offenbart, die für die Bindung und den nachfolgenden Nachweis von Prionen geeignet sind.

Prionen sind normale zelluläre Proteine (PrPc), die vor allem auf der Oberfläche von Neuronen nachgewiesen werden. Durch die Umwandlung des normalen zellulären Proteins in seine abnorme Isoform (PrPsc) resultiert eine pathogene Form mit neuen Eigenschaften. Im Gegensatz zu der normalen Form ist die entstandene Form nicht wasserlöslich und kann durch Proteasen nicht abgebaut werden. Nach der sogenannten "Protein only"-Hypothese führt das Zusammentreffen von pathlogischen und normalen Prionen dazu, dass das normale Molekül seine Konformation ändert und die pathologische Form annimmt. Dieser Prozeß führt zur Akkumulation dieser Proteine im Gehirn und schließlich zu Enzephalopathien, zu denen die Traberkrankheit (Scrapie) des Schafs, die bovine spongiforme Enzephalopathie (BSE) beim Rind und die Creutzfeldt-Jakob-Krankheit (CJD) beim Menschen zählen.

Glykosaminoglycane (GAGS) sind lineare Heteropolysaccharide, die auf Zelloberflächen verankert und eine wesentliche Rolle bei der Zelladhäsion und Migration spielen. Möglicherweise werden Prionen auch über diese Moleküle intrazellulär eingeschleust.

Pentosan (Poly-b-Xylose-2,3-Disulfonat; Pentosanpolysulfat) ist ein polysulfatiertes Polyglykosid (Molekulargewicht 4.000 bis 12.000 Dalton). Dieses Molekülscheint die Akkumulation von Prionen zu inhibieren. Es wurde gezeigt, dass diese Substanz im Vergleich zu anderen sulfatierten GAGS wie Heparin, Heparansulfat und Chondroitinsulfat am stärksten die Produktion von Prionen inhibiert. Diese Inhibition ist möglicherweise auf eine direkte Verbindung mit den Prionen zurückzuführen [B. Caughey and G. Raymond, Journal of Virology, Feb. 1993, p. 643-650; B. Caughey et al., Journal of Virology, Apr. 1994, p.2135-2141; D. Sawitzky, Med. Microbiol Immunol. (1996) 184: 155-161; R. Gabizon et al., Journal of cellular Physiology 157: 319-325 (1993); Show-Ling Shyng et al., Journal of biological Chemistry, 1995 by The American Society for Biochemistry and Molecular Biology, Inc.; C. Farquhar et al., The Lancet, Vol. 353, January 9, 1999]

Es sind bereits PrP-spezifische monoklonale und polyklonale Antikörper hergestellt worden, die beide Varianten PrPc und PrPsc erkennen. Mit Hilfe der verfügbaren monoklonalen Antikörper lassen sich beide Varianten indirekt unterscheiden und somit eine Erkrankung nachweisen.

Dies geschieht bisher durch Versetzung der Untersuchungsproben mit Proteinase K, die nur PrPc zerstört, nicht aber PrPsc. Mit Hilfe der Elektrophorese werden beide Proteine getrennt und die pathologische Form mit Antikörpern nachgewiesen. Obwohl die Sensitivität und Spezifität dieser Tests hoch ist, können nicht alle BSE-Fälle erkannt werden, da ein positives Ergebnis in diesen Tests von der Gesamtmenge der für die Untersuchung eingesetzten Prionen abhängig ist. Eine ausreichen hohe Gesamtmenge ist aber in aller Regel erst im Endstadium der Erkrankung im Gehirngewebe vorhanden, so daß entsprechende Untersuchungen bisher nur an Gewebe aus toten Lebewesen standardmäßig durchgeführt werden können [R. Meyer, Dt. Ärzteblatt 97, Heft 49, 08.12.2000, Seite A-3314]. Außerdem sind diese auf einer Elektrophorese beruhenden Tests langwierig und teuer.

Kürzlich ist es gelungen, mit Hilfe von organischen Lösungsmitteln und einer speziellen Chromatografie die Erreger auch im Blut von Schafen und Elchen aufzuspüren und mit einem Westemblot-Test nachzuweisen [M. Schmerr et al., Journal of Chromatography A, 853 (1999) 207214]. Ein solcher Test ist allerdings extrem langwierig und teuer und in der Praxis als Routinetest kaum zu verwirklichen.

Von Adriano Aguzzi et al. wurde vor kurzem beschrieben, daß PrPsc spezifisch an Plasminogen binden. [M. B. Fischer et al., NATURE, Vol. 408, 23. November 2000] Ein auf dieser Bindung beruhender Test wurde nicht vorgestellt.

Spezifische Labormarker für diese Erkrankungen konnten bisher also nicht gefunden werden, und die Diagnose gelingt in der Regel erst nach dem Tod des Patienten oder nach dem Schlachten des Tieres. [S. B. Prusiner, PNAS USA, Vol. 95, pp. 13363-13383, Nov. 1998; A. L. Horwich and S. Weissman, Cell, Vol. 89, 499-510, May 1997]

In der DE 197 30 132 A1 und der WO 00/29850 werden *in-vitro*-Verfahren zum Nachweis von BSE-Erregern in einer Probe beschrieben, wobei jedoch der Nachweis im Gegensatz zu dem erfindungsgemäßen Verfahren direkt mit Hilfe von immobilisierten Antikörpern durchgeführt wird. Eine Verwendung von Glycosaminoglycan wird nicht beschrieben.

Bis zur vorliegenden Erfindung wurde noch kein Verfahren kommerziell verwirklicht, mit Hilfe dessen eine etwaige BSE-Erktankung an lebenden Organismen nachzuweisen wäre. Gerade dies wäre aber angesichts der zur Zeit bestehenden Unheilbarkeit dieser Erkrankung und der nachgewiesenen Übertragbarkeit der Erkrankung von infizierten Tieren (z.B. Rindern), bei denen die Erkrankung jedoch noch nicht ausgebrochen sein muß, auf den Menschen über die aufgenommene Nahrung für die flächendeckende Prophylaxe und auch für die erfolgreiche Ausmerzung der Erkrankung von überragender Bedeutung. Nicht unerwähnt bleiben sollen in diesem Zusammenhang auch die durch diese Erkrankung angerichteten volkswirtschaftlichen Schäden in Milliardenhöhe.

Trotz des sich aus dem vorstehend Genannten zwangsläufig ergebenden extrem hohen Bedarfs nach einem BSE-Test, der die genannten Nachteile überwindet, konnte ein solcher bis zu vorliegender Erfindung nicht zur Verfügung gestellt werden.

Angesichts des genannten Stands der Technik liegt der vorliegenden Erfindung daher die Aufgabe zugrunde, ein Verfahren zur Verfügung zu stellen, mit dem BSE-Erreger (PrPsc) spezifisch nachgewiesen werden können. Dieses Verfahren soll einfach und schnell in der Handhabung und sicher und kostengünstig in der Durchführung sein sowie einen extrem sensitiven Nachweis ermöglichen. Weiterhin soll das Verfahren *in-vitro* an Proben mit geringer PrPsc -Konzentration durchgeführt werden können, die z.B. aus lebenden Organismen gewonnen werden können.

Gelöst wird diese sowie weitere, nicht explizit genannte Aufgaben, die jedoch aus den hierin einleitend diskutierten Zusammenhängen ohne weiteres ableitbar oder erschließbar sind, durch ein Verfahren mit allen Merkmalen des Patentanspruchs 1. Zweckmäßige Abwandlungen des erfindungsgemäßen Verfahrens werden in den auf Anspruch 1 rückbezogenen Unteransprüchen unter Schutz gestellt.

Dadurch daß man bei einem *in vitro*-Verfahren
(i) eine Probe mit Proteinase K behandelt,
(ii) die so behandelte Probe mit einem bindenden Prinzip, welches an einen festen Träger gebunden ist, in Kontakt bringt,
(iii) das an den festen Träger gebundene bindende Prinzip von der Probe trennt, und
(iv) den gegebenenfalls an das bindende Prinzip gebundene, aus der Probe stammende BSE-Erreger (PrPsc) nachweist, wobei
(v) das bindende Prinzip (ii) ausgewählt ist aus der Gruppe bestehend aus: ein Glykosaminoglycan, Fibronectin oder Lipoprotein A.
   gelingt es auf nicht ohne weiteres vorhersehbare Weise ein Verfahren zum Nachweis von BSE-Erregern in einer Probe zur Verfügung zu stellen, welches diesen Nachweis auf einfache Art und Weise ermöglicht. Dabei weist dieses Verfahren die oben genannten Vorteile gegenüber dem Stand der Technik auf.

Insbesondere ist es
- einfach und schnell in der Handhabung und
- sicher und kostengünstig in der Durchführung,
   und es kann
- an Proben, die aus lebenden Organismen stammen, wie Blutproben, Gewebeproben oder Körperflüssigkeiten wie Urin, Milch, Liquor oder Speichel bzw.
- an Boden- oder Pflanzenproben durchgeführt werden.

Erfindungsgemäß werden BSE-Erreger nachgewiesen. Hierunter wird die pathologische Form des Prion-Proteins (PrPsc) verstanden.

Zum Abbau des nicht-pathologischen Prion-Proteins (PrPc) wird die Probe mit Proteinase K versetzt. Es ist aus dem Stand der Technik bekannt, daß Proteinase K die nicht-pathologische Form hydrolytisch spaltet, die pathologische Form des Prion-Proteins aber nur zu einem geringen Teil. In Anbetracht der vorliegenden Erfindung ist es für den Fachmann jedoch klar, daß auch andere Proteasen, die vorgenanntem Zweck genügen, erfindungsgemäß eingesetzt werden können, ohne daß der Umfang der vorliegenden Erfindung verlassen wird. Die Verfahren zur hydrolytischen Spaltung mit Hilfe der Proteinase K sind dem Fachmann gut bekannt. Insbesondere gibt es eine Reihe von Arbeitsvorschriften in Laborhandbüchern, in denen die verwendbaren Puffersysteme, die anzusetzenden Inkubationszeiten sowie weitere einzuhaltende Reaktionsbedingungen ausführlich beschrieben sind.

Der hydrolytische Abbau des nicht-pathologischen Prionproteins mit Proteinase K wird beispielsweise in M. Schmerr et al., Journal of Chromatography A, 853 (1999) 207-214 beschrieben.

Beispielsweise können die PrPc abgebaut werden, indem eine Probe einem 30minütigen Abbau durch Proteinase K in einer 250 µg/ml Proteinase K-Lösung in einem üblichen Tris/EDTA-Puffer, pH 7,4, 37°C unterzogen werden.

Die nicht hydrolytisch gespaltenen Prion-Proteine, die erfindungsgemäß die pathologische Form PrPsc darstellen, werden mittels eines bindenden Prinzips aus der Probe entfernt. Dieses bindende Prinzip ist vorzugsweise ein Glykosaminoglycan, besonders bevorzugt Pentosan Polyphosphat. Weitere bevorzugte, erfindungsgemäß einsetzbare Glykosaminoglycane sind Heparansulfat, Heparin, Hyaluronsäure, Chondroitinsulfat, Dermatansulfat, Keratonsulfat oder Kongorot. Ebenso erfindungsgemäß bevorzugt einsetzbar ist ein künstlich hergestelltes sulfatiertes Glycosaminoglycan. Weiterhin bevorzugt ist Fibronectin als bindendes Prinzip einsetzbar. Weiterhin bevorzugt ist Lipoprotein A als bindendes Prinzip einsetzbar.

Dieses bindende Prinzip wird erfindungsgemäß an einen festen Träger gebunden.

Als feste Träger kommen hier bevorzugt alle festen Trägermittel in Betracht, die dem Fachmann auf dem Gebiet der Affinitätschromatographie bekannt sind. Besonders bevorzugt werden erfindungsgemäß sogenannte Beads verwendet, d. h. meist kugelförmige Microcarrier aus unterschiedlichen Materialien.

Weitere feste Träger, die erfindungsgemäß bevorzugt verwendet werden können, sind solche Säulenmaterialien, die bereits in der Affinitätschromatographie weite Verbreitung gefunden haben.

Weiterhin bevorzugt erfindungsgemäß einsetzbar sind mit dem oben beschriebenen bindenden Prinzip beschichtete Mikrotiterplatten.

Besonders bevorzugt sind auch Europium (Eu) enthaltende Nanobeads der Firma Seradyn, Indianapolis, USA. Diese Partikel sind mit Europiurn-Chelaten "aufgesaugt", so dass die Oberfläche frei für Kopplungsreaktionen bleibt und die Europium-Chelate nicht auslaufen. Europium-Chelat: tris-(naphtyltrifluorobutandion)-Eu. Wenn diese Partikel mit UV-Licht (333 nm Maximum) erregt werden, emitieren sie eine Lichtausstrahlung bei 613 nm mit einer Zeitdauer von ungefähr 0,5 Millisekunden, was 10 000fach bis 100 000fach länger als die Emissionsdauer der meisten Fluorophore ist. Diese extrem lange Emissionsdauer und die große Stokes-Verschiebung (Differenz zwischen Emissions- und Erregungswellenlänge) erlaubt ihre Verwendung in den auf zeitverzögerter Fluoreszenz basierten Tests. Jedes Partikel enthält >30 000 Europium-Atome, eingeschlossen in tris-Naphtyltrifluorobutandion (ein - Diketon). Für die 100 nm großen Partikel ist der s.g. Quantumertrag (quantum yield, engl.) equivalent zu circa 3 000 Molekülen Fluorescein (einer der meist verwendeten Fluorophore). Das Phycobiliprotein (wahrscheinlich die am stärksten fluoreszierende bekannte Verbindung) hat zum Vergleich einen Quantumgewinn, der dem von ca. 30 Fluorescein Molekülen entspricht. Da ein 100 nm Partikel einen 10 mal größeren Durchmesser im Vergleich zu dem Phycobiliprotein und ein 1 000 mal größeres Volumenmasse Verhältnis hat, weisen diese Beads eine 100 mal höhere Fluoreszenz auf molarer Basis auf, als das Phycobiliprotein.

Die Nanopartikel werden zuerst bestrahlt (s.g. Erregungswellenlänge- bei Europium 340 nm) und nach einer Verzögerung von 400 µs (in diesem Fall) werden die Signale 400 µs lang gemessen. Durch diese Verzögerung wird die Registrierung von unspezifischen kurzdauernden Signalen aus der Matrix vermieden. Die Ergebnisse werden in RFU (relative fluorescence units) dargestellt. Die Messung läuft automatisch mit einem standardisiertem Fluorimeter, mit dem die zeitverzögerte Fluoreszenz gemessen werden kann.

Mithilfe des erfindungsgemäßen Testsystems werden Untersuchungen an Proben, die aus lebenden Organismen stammen, wie Blutproben, Gewebeproben oder Körperflüssigkeiten wie Urin, Milch, Liquor oder Speichel erfolgreich auf die Arrwesenheit von BSE-Erregern (PrPsc; pathologische Form des Prion-Proteins) durchgeführt.

Ebenfalls können Untersuchungen an Boden- oder Pflanzenproben durchgeführt werden.

Besonders bevorzugt wird lysiertes thrombisches Ausgangsmaterial als Probe eingesetzt. Hierfür wird eine Blutprobe zur Gerinnung gebracht, und das entstehende thrombische Material abgetrennt. Das thrombische Material wird durch Hydrolyse mit einer Proteinase abgebaut, und die resultierende Probe direkt im Test eingesetzt.

Der an das bindende Prinzip gebundene BSE-Erreger wird bevorzugt über spezifische Antikörpertests nachgewiesen. Auch diese Tests und Nachweisverfahren sind dem Fachmann gut bekannt. Beispielhaft genannt werden hier ELISA-, und RIA-Verfahren sowie Agglutinationstests und die Durchflusszytometrie.

Prinzipiell sind hier alle Verfahren einsetzbar, die den Nachweis über einen spezifischen Erst-Antikörper, gegebenenfalls unter Verwendung eines für den Erstantikörper spezifischen Zweitantikörpers, erlauben. Verfahren, die ein Verstärkungssystem wie ein an den Erst- oder Zweitantikörper gekoppeltes Enzym aufweisen, sind dabei erfindungsgemäß besonders bevorzugt einsetzbar.

Spezifische Antikörper wurden bereits beschrieben und können u.a. von der Fa. Prionics AG, Universität Zürich, Schweiz, käuflich erworben werden.

Ein erfindungsgemäß besonders bevorzugtes System für den Nachweis des an das bindende Prinzip gebundene PrPSc umfasst an Nanopartikel mit einem Durchmesser von 10-100 nm kovalent gebundene Antikörper. Die Nanopartikel enthalten Ewopium. Mittels der zeitverzögerten Floureszens lassen sich diese Partikel und damit indirekt die Antikörper bzw, die Bindung der Antikörper an PrPsc oder an den Erstantikörper hochempfindlich nachweisen.

Weiterhin ist es bevorzugt, wenn die Antikörper des vorhergehenden Absatzes nicht kovalent, sondern über das dem Fachmann gut bekannte Biotin/Streptavidin-System, wobei bevorzugt die Antikörper biotinyliert vorliegen, an das Nanobead gebunden sind.

Ein weiteres bevorzugtes Ausführungsbeispiel der vorliegenden Erfindung ist es, daß für den Nachweis der Träger markiert vorliegt, insbesondere in Form einer Eu-Markierung (EU-Nanobeads), und nach Abreicherung aus der Probe mittels Bindung an ein zweites bindendes Prinzip, insbesondere einen spezifischen anti-PrPsc-Antikörper die entsprechende Markierung durch entsprechenden Nachweis, insbesondere die zeitverzögerte Floureszens, nachgewiesen wird.

Es ist der vorliegenden Erfindung zu eigen, daß die einzelnen Bestandteile des erfindungsgemäßen Nachweisverfahrens dem Fachmann bekannt sind. Es war jedoch in keiner Weise vorherzusehen, daß eine Kombination dieser einzelnen Schritte zu einem Verfahren führt, daß in so überaus überraschend günstiger

Weise für ein solchermaßen intensives Bedürfnis der Fachwelt eine einfache Lösung bietet.

Die nachfolgenden Beispiele erläutern die Erfindung näher. Sie sollen jedoch keineswegs limitierend verstanden werden.

### BEISPIEL 1

### Bindung von Pentosan an Microcarrier

Als Träger wurde Toyopearl HW55 (kommerziell erhältliches kreuzvernetztes Polyacrylat-Gel der Toyo Soda Manufacturing Co. Ltd., mit einer Partikelgröße von 50-100 µm) verwendet.

In 10 ml des Gels wurden 6 ml einer gesättigten wässrigen NaOH-Lösung und 15 ml Epichlorhydrin gegeben, und das Reaktionsgemisch wurde bei 50°C zwei Stunden unter Rühren inkubiert. Das Gel wurde aufeinanderfolgend mit Alkohol und Wasser gewaschen, um Epoxy-Gruppen in das Gel einzuführen. Zu dem resultierenden Epoxy-aktivierten Gel wurden 20 ml konzentriertes wässriges Ammoniak zugefügt, und das Reaktionsgemisch wurde 2 Stunden bei 50°C gerührt, um in das Gel Amino-Gruppen einzuführen.

3 ml des so erhaltenen aktivierten Gels, welches Amino-Gruppen enthielt, wurde zu 10 ml einer200 mg Pentosan Polysulfat enthaltenden wässrigen Lösung (pH 4,5), hinzugefügt. Zu dem resultierenden Reaktionsgemisch wurden 200 mg 1-Ethyl-3-(dimethylaminopropyl)-carbodiimid hinzugefügt, wobei der pH-Wert des Reaktionsgemisches auf 4,5 eingestellt wurde, und das resultierende Reaktionsgemisch wurde 24 Stunden bei 4°C geschüttelt. Nach Vervollständigung der Reaktion wurde das resultierende Reaktionsgemisch aufeinanderfolgend mit einer 2 M wässrigen NaCL Lösung, einer 0,5 M wässrigen NaCL Lösung und Wasser gewaschen, wodurch das gewünschte Gel erhalten wurde, auf dem Pentosan Polyphosphat immobilisiert war.

### BEISPIEL 2

Prionenhaltige Testproben werden nach üblichen Methoden mit Proteinase K zur Elimination der normalen Prionproteine PrPc behandelt.

Dazu wird die Probe auf eine Konzentration von 50 µg/ml Proteinase K (Boehringer) eingestellt, und 1 Stunde bei 37°C inkubiert. Die näheren Bedingungen dieses enzymatischen Abbaus sind bei Schmerr et al., supra, beschrieben.

Beispielsweise kann folgendes Protokoll verwendet werden:
50 µl 1 mg/ml Proteinase K (Sigma, Kat.Nr. 82456) werden in 1 ml Probe pipettiert.
30 Min bei 37°C wird inkubiert.
100 µl 4 mM Pefabloc SC PLUS (Roche; Kat.Nr. 1 873 601) werden zugegeben.

Dann wird 5 Min bei Raumtemperatur inkubiert.

Der Überstand wird mit Pentosan Polyphosphat beschichteten Beads aus Beispiel 1 versetzt. Es hat sich hier gezeigt, daß die Anwesenheit von 1 mM Zink die Bindung fördert.

Die Beads werden durch Zentrifugation in einer Hettich-Tischzentrifunge sedimentiert und von der Testprobe getrennt.

Anschliessend werden die Beads mit spezifischen Antikörpern gegen Prionen (Prionics AG, Univerität Zürich, 8057 Zürich, Schweiz) inkubiert und im ELISA mit Hilfe eines sekundären Antikörpers getestet. Ein positiver Testansatz ist gekennzeichnet durch vermehrte Anlagerung des sekundären Antikörpers an den Ligandenkomplex, die wiederum über die Umsetzung eines farblosen Substrates durch das an den Zweitantikörper gebundene Enzym gemessen werden kann.

Im Blut infizierter Tiere konnte der BSE-Erreger so auf erstaunlich einfache Weise nachgewiesen werden.

### BEISPIEL 3

Statt eines Elisa-Tests wurde ein Agglutinationstest durchgeführt.

Auch auf diese Weise konnte das BSE-Erreger auf erstaunlich einfache Weise nachgewiesen werden.

### BEISPIEL 4

Statt eines Elisa-Tests wurden die mit Zweitantikörper markierten Beads vermittels Durchflusszytometrie nachgewiesen.

Auch auf diese Weise konnte das BSE-Erreger auf erstaunlich einfache Weise nachgewiesen werden.

Diese Vorgehensweise zum Nachweis von Prionen ist völlig neu und praktikabel. Mit dieser Methode können Blutproben, Gewebeproben, Körperflüssigkeiten (z. B. Urin, Milch, Liquor, Speichel, etc.) tierischer, menschlicher und pflanzlicher Herkunft getestet werden. Auch Bodenproben können auf eine Kontamination getestet werden.

### BEISPIEL 5

Zur kovalenten Bindung von Antikörpern an Nanobeads (FluoroMax™ Fluorescent microparticles, Seradyn, Indianapolis, USA) werden diese mit Carbodiimid und Hydroxysuccinimid behandelt, um eine sichere und feste Bindung zwischen den Carboxylgruppen auf der Partikeloberfläche und den Proteinmolekülen (Antikörpern) zu gewährleisten. So bleiben die Antikörper mit den Nanopartikel stabil gebunden (längere Haltbarkeit des Konjugats). Die Antikörper lassen sich nicht von den Beads bei den nachfolgenden Behandlungen (verschiedene Reaktionspuffer) trennen.

### BEISPIEL 6

Nachweis der Bindung des PrPsc an Pentosanpolyphosphat mittels Antikörper-Europium-Nanobead-Komplexe.

Die Europium-Nanopartikel wurden von der Firma Seradyn, Indianapolis, USA bezogen. Diese Partikel sind mit Europium-Chelate "aufgesaugt", so dass die Oberfläche frei für Kopplungsreaktionen bleibt und die Europium-Chelate nicht auslaufen. Europium-Chelat: tris-(naphtyltrifluorobutandion)-Eu. Wenn diese Partikel mit UV-Licht (333 nm Maximum) erregt werden, emitieren sie eine Lichtausstrahlung bei 613 nm mit einer Zeitdauer von ungefähr 0,5 Millisekunden, was 10 000fach bis 100 000fach länger als die Emissionsdauer der meisten Fluorophore ist. Diese extrem lange Emissionsdauer und die große Stokes-Verschiebung (Differenz zwischen Emissions- und Erregungswellenlänge) erlaubt ihre Verwendung in den auf zeitverzögerter Fluoreszenz basierten Tests. Jedes Partikel enthält >30 000 Europium-Atome, eingeschlossen in tris-Naphtyltrifluorobutandion (ein -Diketon). Für die 100 nm großen Partikel ist der s.g. Quantumertrag (quantum yield, engl.) equivalent zu circa 3 000 Molekülen Fluorescein (einer der meist verwendeten Fluorophore). Das Phycobiliprotein (wahrscheinlich die am stärksten fluoreszierende bekannte Verbindung) hat zum Vergleich einen Quantumgewinn, der dem von ca. 30 Fluorescein Molekülen entspricht. Da ein 100 nm Partikel einen 10 mal größeren Durchmesser im Vergleich zu dem Phycobiliprotein und ein 1000 mal größeres VolumenlMasse Verhältnis hat, weisen diese Beads eine 100 mal höhere Fluoreszenz auf molarer Basis auf, als das Phycobiliprotein.

Die Nanopartikel werden zuerst bestrahlt (s.g. Erregungswellenlänge- bei Europium 340 nm) und nach einer Verzögerung von 400 µs (in diesem Fall) werden die Signale 400 µs lang gemessen. Durch diese Verzögerung wird die Registrierung von unspezifischen kurzdauernden Signalen aus der Matrix vermieden. Die Ergebnisse werden in RFU (relative fluorescence units) dargestellt. Die Messung läuft automatisch mit einem standardisiertem Fluorimeter, mit dem die zeitverzögerte Fluoreszenz gemessen werden kann. Beschrieben ist dieses System u.a. bei Ci, Y., *et al.,* J. Immun. Meth., 179, 233-241, 1995 sowie Souka *et al.* Clin. Chem., 47:3, 561-568.

Für ähnliche Systeme (Eu-Nanobeads-Konjugate) werden Detektionsgrenzen für Prostata-spezifische-Antigene von 7,3 x 10⁵ Molekülen/ml berichtet (für Prionprotein würde das ca. 36,3 fg entsprechen). Die bisher beschriebenen Zeit Nachweismethoden können 50 pg Prionprotein detektieren (Barnard, G. *et al*., Luminescence, 15:6, 357-362, 2000)

### BEISPIEL 7

### Antikörperkoppelung an Träger mit dem Biotin/Streptavidin-System

Biotin wird in den so genannten Zweischritt-Techniken im Zusammenhang mit konjugiertem oder immobilisiertem Strept(Avidin) verwendet. Die Bindung des Biotins an das Strept(Avidin) ist sehr schnell und stabil. Es sind verschiedene Techniken zum Biotinylieren von Antikörpern beschrieben. Außerdem gibt es auch Kits von mehreren Firmen mit dem entsprechenden Protokoll. Normalerweise wird das Biotin durch die Primäramine der Proteine (z.B. Lysin) konjugiert und so werden 3 bis 6 Moleküle Biotin pro Protein-Molekül gebunden. Beispielsweise kann das Sulfo-NHS-LC-Biotin (Pierce) verwendet werden. Die Trennung des nicht konjugierten Biotins vom Antikörper kann mit Zentrifugenmikrokonzentratoren Nanosep™ (Pall) durchgeführt werden. Die Stufe des Biotinilierens wird durch die s.g. HABA-Reaktion (HABA-2-(4'hydroxyazobenzene)-benzoic acid) mit Überschuß von Avidin photometrisch bestimmt.

Die Beschichtung der Nanopartikel mit Streptavidin wird nach Härmä *et al.,* Clin. Chem., 47:3; 561-568 (2001) durchgeführt. Nach Vorwaschen der Beads mit PBS, pH 7,0 mit Nanosep™ Zentrifugenmikrokonzentratoren, werden diese in demselben Puffer mit Ultraschallsonde resuspendiert. Danach erfolgt die Aktivierung der Carboxylgruppen mit 10 mM N-(3 -dimethylaminopropyl)-N'-ethylcarbodiimide (EDAC), (Pierce) und N-hydroxysulfosuccinimide (NHS), (Sigma) für 30 Minuten. Nach zwei Waschprozeduren (wie oben, aber mit Carbonatpuffer pH 9,0) wird 15 µM Streptavidin zugegeben und die Beads für 1 Stunde inkubiert. Am Ende werden die Nanopartikel 5 mal mit 2 mM Tris-HCl (Sigma), pH 7,0 gewaschen und bei 4°C gelagert.

### BEISPIEL 8:

### Bindung von PrPSc an Europium-Nanobeads über Fibronectin (s. Beispiel 7)

Es hat sich hier gezeigt, daß es insbesondere wichtig ist, eine Methode zu verwenden, bei der die Konformation der Fibronectin-Moleküle am geringsten beeinflusst wird.

Das Abtrennen der (Nano-)Beads von der Probe kann durch Zentrifugation und/oder durch Verwendung sogenannter Zentrifugenkonzentratoren (Mikrofilter) erfolgen. Je nach Bindungsstärke zwischen Fibronektin und PrPsc können die Beads- PrPsc-Komplexe gewaschen werden, um möglichst höchste Trennung der beiden PrP-Formen zu bekommen.

### Das prinzipielle Vorgehen war wie folgt:

1. Fibronectin wird mit Nanobeads (Europium-Nanobeads) gekoppelt.
2. Die Probe wird mit Fibronectin-Nanobeads inkubiert. Die pathologische Form des PrP (PrPSc) haftet an dem Fibronectin und die normale Form (PrPC) nichtbleibt in dem Reaktionsgemisch aufgelöst.
3. Nachher werden die Fibronectin-Nanobeads durch Abzentrifugieren von der Probe getrennt und in einem Reaktionspuffer resuspendiert.
4. Die resuspendierten Fibronectin-Nanobeads werden auf einer mit monoklonalen Antikörper (gegen PrP) beschichteten Mikrotiterplatte inkubiert (Bildung des Komplexes MoAk-PrPSc-Fibronectin-Nanobeads).
5. Nach dem Waschvorgang wird die Fluoreszenz gemessen (zeitaufgelöst) und die Konzentration von PrPSc bestimmt.

### Eine detaillierte Anweisung folgt:

- Human FN (FIBRONECTIN- Lot: 000832, Firma: BD Biosciences. MG: 440 kDa) oder andere Fibronectinrekombinante.
- 1 mg/ml FN: Eine Flasche 1 mg FN wird zu RT gebracht und wird mit 1 ml sterilem dest. H₂O für 30 Min aufgelöst.
- 0,5 ml 1% MP (Seradyn, FLUORO-MAX FLUORESCENT PARTTCLES PartNr. 1347-0350) mit 0,5 ml 50 mM pH 6,1 MES (Morpholinoethansulfonsäure, Sigma- ProduktNr. M8250) und Nanosep 300kDa cut-off (Pall Life Sciences; ProduktNr. OD300C33) 5 min bei 14 000g 2x waschen.
- Nach dem letzten Waschvorgang in 0,5 ml 50 mM pH 6,1 MES resuspendiren, in 2,0 ml Eppendorfröhrchen überführen und bis 1,0 ml mit 50 mM pH 6,1 MES zufullen.

### Voraktivierung:

a) EDAC (Carbodiimid- Fa. Pierce. Kat.Nr. 22980): 0.6 molarer Überschuß: 0,5 ml 1% MP= 5 MP; MP acid content meq/g = µmol/mg;
b) (acid content, µmol/mg) (5 mg MP) (gewünschtes Verhältnis) = µmol EDAC benötigt: 0,1566 x 5 x 0,6 = 0,4698 µmol EDAC benötigt
c) (µmol EDAC benötigt) / (52 µmol/ml) = ml EDAC Stammlösung pro ml Reaktion: 0,4698/52= 0,009 ml EDAC Stammlösung
d) EDAC Stammlösung: 10 mg/ml in dest. Wasser, unmittelbar vor der Verwendung anfertigen.
e) In folgender Reihe zu den gewaschenen MP pipettieren:
   - 230 µl NHS Stammlösung (50 mg/ml in dest. Wasser N-hydroxysuccinimid-Sigma, Kat.Nr. H7377)
   - 9 µl EDAC Stammlösung
   - Bei RT (Raumtemperatur) 30 Minuten bei ständigem Mischen inkubieren.
   - Die MP 2x waschen mit 50 mM pH 6,1 MES und Nanosep wie in 3.
   - Nach dem letzten Waschvorgang LWV in 0,5 ml 100 mM pH 6,1 MES resuspendiren und in 2,0 ml Eppendorf überführen.
   - 0,5 ml FN-Lösung von 2. zugeben, gut mischen.
   - 2 St bei RT bei ständigem mischen inkubieren.
   - MP 2x mit 50 mM pH 6,1 MES und Nanosep waschen wie in 3.
   - Nach dem LWV in 0,5 ml MNTA (25 mM MOPSO Sigma Kat.Nr.M8389, pH 7,4; 100 mM NaCl-Sigma, Kat. Nr. 71378; 0,1% Tween20 Sigma, Kat.Nr 27,434-8; 0,1% NaN3 Sigma Kat.Nr. 71290) resuspendieren.
   - Bei 4°C aufbewahren.

### Beschichten der Mikrotiterplatte:

- Platte (Nunc, Fluoro-Nunc Modules F 16 Maxi-Sorp, Kat.Nr. 47515) coaten:

- 6H4 (Monoklonaler Antikörper, Prionics): 8µg/ml, 96 wells -in Bic/Carb Puffer pH 9,4 (Pierce, Kat.Nr. 28382) verdünnen, 50 µl/well pipettieren.
- 3 Stunden bei 37°C inkubieren.

### Blockieren unspezifischer Bindestellen:

- 1x200 µl mit Waschpuffer (5 mM Trizma pH 7,8 Sigma Kat.Nr. 93349; 150 mM NaCl; 0,05% Tween20) waschen.
- 100 µl Blocking Puffer(50 mM Trizma pH 7,8 Sigma Kat.Nr. 93349; 150 mM NaCl; 2% Rinder Serumalbumin, Immucore Kat.Nr. 004410; 2 Gelatine Sigma Kat.Nr. G7765; 0,05% Tween20; 0,05% NaN3) je Kavität pipettieren.
- 1 Stunde bei Raumtemperatur schütteln -450 rpm.
- Absaugen des Überstandes.

### Antigen-MP-FN Inkubation:

- Die MP-FN 1: 742 in PBS-Tween20 2% ( PBS Pierce Kat.Nr. 28374; Tween20 2%) verdünnen.
- In Eppendorfröhrchen 50 µl Proteinase K behandelte Probe und 50 µl verdünnte MP-FN pipettieren und 2 St bei RT bei ständigem Mischen inkubiren.
- 2 Min bei 14 000g mit Nanosep zentrifugieren.
- Filtrat verwerfen und die MP-FN in 100 µl PBS-Tween20 2% resuspendieren.
- 30 µl je Probe exakt auf Kavitätenboden.
- 2 Stunden bei 37°C bei 450 rpm inkubieren.
- 2x300 µl PBS-Tween20 2% waschen.

### Messen (zeitverzögerte Floureszens):

- Gerät: Tecan GENios, Program XFluor4: 10 Blitze, Verzögerung 400 µs, Integration: 400 µs.

### BEISPIEL 9

Fibronectin wird mit Beads (Polystyrenbeads) gekoppelt:

Proteinase K behandelte und unbehandelte Proben werden mit Fibronectin-Nanobeads inkubiert

Dann wird die Probe mit den Fibronectin-Beads inkubiert.

Nachher werden die Fibronectin-Beads gewaschen und in einem Reaktionspuffer resuspendiert.

Die resuspendierten Beads werden mit einem monoklonalen fluoreszenzmarkierten Antikörper (gegen PrP) inkubiert.

Nach der Inkubation werden die Fluoreszenzsignale in einem Durchflußzytometer gemessen.

### BEISPIEL 10:

Bindung von PrPSc in mit Fibronectin beschichteten Mikrotiterplatten.
1. Die Mikrotiterplatte wird mit Fibronectin beschichtet (gecoatet).
2. Die Probe wird in der Mikrotiterplatte inkubiert. Die pathologische Form des PrP (PrPSc) haftet an dem Fibronectin und die normale Form (PrPC) nichtbleibt in dem Reaktionsgemisch aufgelöst.
3. Nach dem Waschvorgang werden die mit monoklonalen Antikörper (gegen PrP) gekoppelten Europium-Nanobeads in die Mikrotiterplatte pipettiert und inkubiert.
4. Nach dem Waschvorgang wird die Fluoreszenz gemessen (zeitaufgelöst) und die Konzentration von PrPSc bestimmt.

Ein detailliertes Verfahren lautet beispielsweise wie folgt:

### Platte (Nunc, Fluoro-Nunc Modules F16 Maxi-Sorp, Kat.Nr. 47515) coaten:

- FN 2,5 µg/ml in PBS (Pierce, Kat.Nr. 28374) pH 7,2 verdünnen, 50 µl/well pipettieren.
- 1 St bei RT inkubieren.
- Absaugen.
- Platte vorsichtig mit dest. Wasser 3x 300 µl waschen.

### Antigen Inkubation:

- 30 µl Proteinase K behandelte Probe in die Kavitäten pipettieren.
- 1,5 St bei RT- 450 rpm inkubieren.

### MP-3F4 Inkubation:

• 1x200 µl PBS-Tween20 2% waschen.
• Die MP 1:296 in PBS-Tween20 2% verdünnen.
• Die Verdünnung gut mischen.
**•** 30 µl genau auf Kavitätenboden pipettieren.
• 4 St bei bei 30°C inkubieren- 450 rpm schütteln.
• 2x200 µl mit PBS-Tween20 2% waschen.

### Messen:

- Gerät: Tecan GENios, Program XFluor4: 10 Blitze, Verzögerung 400 µs, Integration: 400 µs.

### BEISPIEL 11:

Bindung von PrPSc an Europium-Nanopoartikel über Lipoprotein A.

Vorgehensweise s. Beispiel 9 (anstelle von Fibronektin wurde Lipoprotein A eingesetzt. Es kann auch Apolipoprotein A eingesetzt werden.).

### BEISPIEL 12:

Bindung von PrPSc an mit Lipoprotein A beschichteten Polystyrenbeads.

Vorgehensweise s. Beispiel 10 (anstelle von Fibronektin wurde Lipoprotein A eingesetzt. Es kann auch Apolipoprotein A eingesetzt werden.).

### BEISPIEL 13:

Bindung von PrPSc in mit Lipoprotein A beschichteten Mikrotiterplatten.

Vorgehensweise s. Beispiel 11 (anstelle von Fibronektin wurde Lipoprotein A eingesetzt. Es kann auch Apolipoprotein A eingesetzt werden.).

## Patentansprüche

1. *In Vitro*-Verfahren zum Nachweis von BSE-Erregern (PrPsc) in einer Probe, wobei
a. die Probe mit Proteinase K behandelt wird
b. die so behandelte Probe mit einem PrPsc-bindenden Prinzip, welches an einen festen Träger gebunden ist, in Kontakt gebracht wird,
c. das an den festen Träger gebundene bindende Prinzip von der Probe getrennt wird, und der gegebenenfalls an das bindende Prinzip gebundene, aus der Probe stammende BSE-Erreger (PrPsc) nachgewiesen wird,
**dadurch gekennzeichnet, daß** das bindende Prinzip ausgewählt ist aus der Gruppe bestehend aus:Glykosaminoglycan, Fibronectin oder Lipoprotein A.

2. Verfahren nach Anspruch 1, wobei das bindende Prinzip Fibronectin oder Lipoprotein A ist, und die Probe vor Durchführung der Stufe b) nicht mit Proteinase K behandelt wird.

3. Verfahren nach Anspruch 1 oder 2, wobei für den Nachweis (c) ein PrPscspezifischer monoklonaler Antikörpertest verwendet wird.

4. Verfahren nach Anspruch 1 oder 2, wobei für den Nachweis (c) der Träger aus Stufe (b) markiert vorliegt, und nach Abreicherung aus der Probe mittels Bindung an ein zweites bindendes Prinzip die entsprechende Markierung durch entsprechenden Nachweis nachgewiesen wird.

5. Verfahren nach einem der vorstehenden Ansprüche, wobei die Probe aus dem menschlichen oder tierischen Körper oder aus Pflanzen stammt.

6. Verfahren nach Anspruch 5, wobei die Probe eine Blutprobe, Gewebeprobe oder eine Körperflüssigkeit wie Urin, Milch, Liquor oder Speichel ist.

7. Verfahren nach Anspruch 5, wobei die Probe lysiertes thrombisches Material ist.

8. Verfahren nach einem der Ansprüche 1 bis 4, wobei die Probe eine Bodenprobe ist.

9. Verfahren nach einem der vorstehenden Ansprüche, **dadurch gekennzeichnet, daß** als fester Träger ein Microcarrier (Bead) verwendet wird

10. Verfahren nach einem der vorstehenden Ansprüche, **dadurch gekennzeichnet daß** der für den Nachweis verwendete Antikörper an ein Nanobead mit einem Durchmesser von 10-100 nm gekoppelt ist, welches Europium enthält, und wobei der gegebenenfalls an das bindende Prinzip gebundene, aus der Probe stammende BSE-Erreger (PrPsc) mit Hilfe der zeitverzögerten Floureszens nachgewiesen wird.

11. Verfahren nach einem der vorstehenden Ansprüche, **dadurch gekennzeichnet daß** die Bindung des PrPsc an das bindende Prinzip nachgewiesen wird über zeitverzögerte Floweszens von an das bindende Prinzip gebundenen, Europium enthaltenden Nanobeads.

12. Verfahren nach einem der vorstehend genannten Ansprüche, **dadurch gekennzeichnet, daß** als fester Träger Mikrotiterplatten verwendet werden.

13. Verfahren nach einem der vorstehend genannten Ansprüche, **dadurch gekennzeichnet, daß** als fester Träger eine Fritte verwendet wird.

14. Verwendung von an einen festen Träger gebundenem Fibronectin oder Lipoprotein A zum Nachweis von Prionen in einem diagnostischen System.

15. Diagnostisches System in Form eines Kits zur Durchführung des Verfahrens zum Nachweis von BSE-Erregern (PrPsc) nach einem der Ansprüche 1-13, mindestens umfassend das an einen festen Träger gebundene bindende Prinzip und eine Proteinase, bevorzug Proteinase K.

16. Diagnostisches System nach Anspruch 15, weiterhin umfassend einen spezifischen Antikörper gegen PrPsc sowie gegebenenfalls einen gegen diesen spezifischen Antikörper gerichteten zweiten Antikörper.

## Claims

1. An in Vitro method for the detection of BSE pathogens (PrPsc) in a sample, whereby
a. the sample is treated with proteinase K,
b. the sample thus treated is brought into contact with a PrPsc-binding principle, which is bound to a solid carrier,
c. the binding principle bound to the solid carrier is separated from the sample, and the BSE pathogen (PrPsc) originating from the sample and bound as the case may be to the binding principle is detected,
**characterised in that** the binding principle is selected from the group comprising: glycosaminoglycan, fibronectin or lipoprotein A.

2. The method according to claim 1, whereby the binding principle is fibronectin or lipoprotein A, and the sample is not treated with proteinase A before step b) is carried out.

3. The method according to claim 1 or 2, whereby a PrPsc-specific monoclonal antibody test is used for detection (c).

4. The method according to claim 1 or 2, whereby the carrier from step (b) is present in tagged form for detection (c), and after depletion from the sample by means of binding to a second binding principle the corresponding tagging is detected by suitable detection.

5. The method according to any one of the preceding claims, whereby the sample originates from the human or animal body or from plants.

6. The method according to claim 5, whereby the sample is a blood sample, tissue sample or a body fluid such as urine, milk, liquor or saliva.

7. The method according to claim 5, whereby the sample is lysated thrombic material.

8. The method according to any one of claims 1 to 4, whereby the sample is a soil sample.

9. The method according to any one of the preceding claims, **characterised in that** a microcarrier (bead) is used as a solid carrier.

10. The method according to any one of the preceding claims, **characterised in that** the antibody used for the detection is coupled to a nanobead with a diameter of 10-100 nm, which contains europium, and whereby the BSE pathogen (PrPsc) originating from the sample and bound as the case may be to the binding principle is detected with the aid of time-delayed fluorescence.

11. The method according to any one of the preceding claims, **characterised in that** the binding of the PrPsc to the binding principle is detected via time-delayed fluorescence of nanobeads containing europium and bound to the binding principle.

12. The method according to any one of the aforementioned claims, **characterised in that** microtitre plates are used as a solid carrier.

13. The method according to any one of the aforementioned claims, **characterised in that** a frit is used as a solid carrier.

14. Use of fibronectin or lipoprotein A bound to a solid carrier for the detection of prions in a diagnostic system.

15. A diagnostic system in the form of a kit for the performance of the method for the detection of BSE pathogens (PrPsc) according to any one of claims 1-13, at least including the binding principle bound to a solid carrier and a proteinase, preferably proteinase K.

16. The diagnostic system according to claim 15, further including a specific antibody against PrPsc and optionally a second antibody directed against this specific antibody.

## Revendications

1. Procédé *in vitro* pour détecter des agents de l'ESB (PrPsc) dans un échantillon, dans lequel :
a. on traite l'échantillon avec de la protéinase K,
b. l'échantillon ainsi traité est mis en contact avec un principe liant le PrPsc, qui est lié à un support solide,
c. on sépare de l'échantillon le principe liant, lié au support solide, et on détecte l'agent de l'ESB (PrPsc) provenant de l'échantillon, et éventuellement lié au principe liant,
**caractérisé en ce que** le principe liant est choisi dans le groupe consistant en le glycosaminoglycanne, la fibronectine ou la lipoprotéine A.

2. Procédé selon la revendication 1, dans lequel le principe liant est la fibronectine ou la lipoprotéine A, et l'échantillon, avant mise en oeuvre de l'étape b), n'est pas traité avec la protéinase K.

3. Procédé selon la revendication 1 ou 2, dans lequel, pour la détection (c), on utilise un test faisant appel à un anticorps monoclonal spécifique du PrPsc.

4. Procédé selon la revendication 1 ou 2, dans lequel, pour la détection (c), le support de l'étape (b) est présent sous forme marquée et, après appauvrissement à partir de l'échantillon, grâce à une liaison à un deuxième principe liant, on détecte par une détection appropriée le marquage correspondant.

5. Procédé selon l'une des revendications précédentes, dans lequel l'échantillon provient de l'organisme humain ou animal, ou de végétaux.

6. Procédé selon la revendication 5, dans lequel l'échantillon est un échantillon sanguin, un échantillon tissulaire ou un liquide corporel tel que l'urine, le lait, le liquide céphalorachidien ou la salive.

7. Procédé selon la revendication 5, dans lequel l'échantillon est un matériel thrombique lysé.

8. Procédé selon l'une des revendications 1 à 4, dans lequel l'échantillon est un échantillon de sol.

9. Procédé selon l'une des revendications précédentes, **caractérisé en ce qu'**on utilise en tant que support solide un micro-support (perle).

10. Procédé selon l'une des revendications précédentes, **caractérisé en ce que** l'anticorps utilisé pour la détection est couplé à une nanoperle ayant un diamètre de 10 à 100 nm, qui contient de l'europium, ce à l'occasion de quoi on détecte à l'aide d'une fluorescence retardée dans le temps l'agent de l'ESB (PrPsc) provenant de l'échantillon et éventuellement lié au principe liant.

11. Procédé selon l'une des revendications précédentes, **caractérisé en ce que** la liaison du PrPsc au principe liant est détectée par l'intermédiaire d'une fluorescence retardée dans le temps de nanoperles contenant de l'europium et liées au principe liant.

12. Procédé selon l'une des revendications précédentes, **caractérisé en ce qu'**on utilise en tant que support solide des plaques de microtitrage.

13. Procédé selon l'une des revendications précédentes, **caractérisé en ce qu'**on utilise une fritte en tant que support solide.

14. Utilisation de fibronectine ou de lipoprotéine A liée à un support solide pour détecter des prions dans un système diagnostique.

15. Système diagnostique sous forme d'un kit pour mettre en oeuvre le procédé de détection d'agents de l'ESB (PrPsc) selon l'une des revendications 1 à 13, comportant au moins le principe liant, lié à un support solide, et une protéinase, de préférence la protéinase K.

16. Système diagnostique selon la revendication 15, comprenant en outre un anticorps spécifique contre le PrPsc, et éventuellement un deuxième anticorps dirigé contre cet anticorps spécifique.
